Europäisches Patentamt

European Patent Office

Office européen des brevets

(11) Veröffentlichungsnummer: **0 262 327**
A2

(12) **EUROPÄISCHE PATENTANMELDUNG**

(21) Anmeldenummer: 87110899.9

(51) Int. Cl.⁴: **G01N 33/18** , G01N 1/20

(22) Anmeldetag: 28.07.87

(30) Priorität: 02.10.86 DE 3633504

(43) Veröffentlichungstag der Anmeldung:
06.04.88 Patentblatt 88/14

(84) Benannte Vertragsstaaten:
AT BE CH DE ES FR GB GR IT LI LU NL SE

(71) Anmelder: **Munk GmbH**

**D-4700 Hamm (Rhynern)(DE)**

(72) Erfinder: **Munk, Karl Heinz**
**Streitland 5**
**D-4700 Hamm 1(DE)**

(74) Vertreter: **Patentanwälte Meinke und**
**Dabringhaus Dipl.-Ing. J. Meinke Dipl.-Ing. W.**
**Dabringhaus**
**Westenhellweg 67**
**D-4600 Dortmund 1(DE)**

(54) **Verfahren und Vorrichtung zur Messung von Schadstoffen in Abwässern.**

(57) Bei der Messung von Schadstoffen in Abwässern soll eine Lösung geschaffen werden, welche sicherstellt, daß die Schadstoffermittlung entsprechend der tatsächlichen Schadstoffbelastung in Abwässern mit einem möglichst geringen, wirtschaftlich noch vertretbaren Aufwand durchgeführt werden kann.

Dies wird dadurch erreicht, daß die Menge des gesamten Abwassers kontinuierlich gemessen und ein bestimmter Teil des jeweils abgeführten Abwassers abgezweigt und gesammelt wird, worauf die Schadstoffmengen in dem abgezweigten und gesammelten Abwasser bestimmt werden und entsprechend dem Verhältnis der Menge des abgezweigten und gesammelten Abwassers zur Menge des gesamten Abwassers die Schadstoffmengen im gesamten Abwasser ermittelt bzw. hochgerechnet werden.

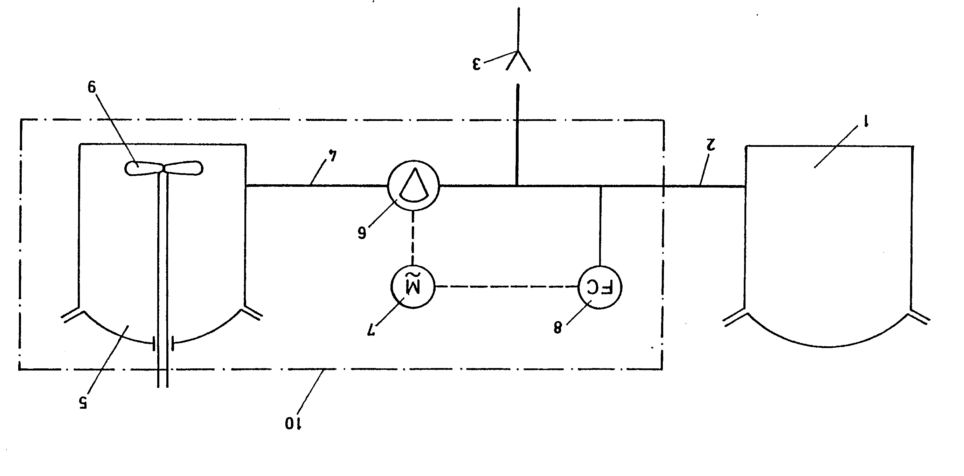

Hierzu ist die einzige Fig. zu veröffentlichen.

## "Verfahren und Vorrichtung zur Messung von Schadstoffen in Abwässern"

Die Erfindung betrifft ein Verfahren zur Messung von Schadstoffen in Abwässern sowie eine zur Durchführung des Verfahrens geeignete Vorrichtung.

Bei vielen Anlagen wie Verzinkereien, Vernickelungs-oder Eloxieranlagen richten sich die Abwassergebühren nach den im Abwasser befindlichen Schadstoffen, deren Bestimmung bisher ganz allgemein durch Kontrollpersonen der Abwasserbehörden in unregelmäßigen Abständen zu vorher nicht angemeldeten Zeiten erfolgt, wobei dann die bei diesen Kontrolluntersuchungen ermittelten Schadstoffwerte einer Hochrechnung für bestimmte Zeiten und daraus sich ergebende Abwassermengen zugrundegelegt werden. Da sich aber nun sowohl die Menge der Abwässer als auch die Konzentrationen der einzelnen Schadstoffe in Anlagen der zuvor erwähnten Art beträchtlich ändern können bzw. diese Konzentrationen der Schadstoffe starken Schwankungen unterworfen sind, kann es vorkommen, daß bei Messungen zu ungünstigen Zeitpunkten sehr ungünstige Gesamtschadstoffmengen für einen größeren Zeitraum der Ermittlung der Abwassergebühren zugrundegelegt werden, was zu ungerechtfertigt hohen Belastungen der betreffenden Betriebe führen kann. Andererseits ist es natürlich auch möglich, daß zu niedrige Schadstoffbelastungen und damit niedrige Abwassergebühren bei nur sporadischen Messungen der Schadstoffe gemäß bisheriger Praxis vorkommen können.

Aufgabe der Erfindung ist die Schaffung einer Lösung, welche sicherstellt, daß die Schadstoffermittlung entsprechend der tatsächlichen Schadstoffbelastung in Abwässern mit einem möglichst geringen, wirtschaftlich noch vertretbaren Aufwand durchgeführt werden kann.

Diese Aufgabe wird gemäß der Erfindung dadurch gelöst, daß die Menge des gesamten Abwassers kontinuierlich gemessen und ein bestimmter Teil des jeweils abgeführten Abwassers abgezweigt und gesammelt wird, worauf die Schadstoffmengen in dem abgezweigten und gesammelten Abwasser bestimmt werden und entsprechend dem Verhältnis der Menge des abgezweigten und gesammelten Abwassers zur Menge des gesamten Abwassers die Schadstoffmengen im gesamten Abwasser ermittelt bzw. hochgerechnet werden.

Die Abzweigung der Menge des zu sammelnden Abwassers zur Menge des gesamten Abwassers kann dabei etwa in einem Verhältnis von 1 : 1000 erfolgen, d.h., daß bei 1 m³ Abwasser jeweils 1 Liter abgezweigt und gesammelt wird, was im allgemeinen zu wirtschaftlich vertretbaren Lösungen hinsichtlich des erforderlichen apparativen Aufwands führt.

Zweckmäßig kann man die Abzweigung des zu sammelnden Abwassers durch eine Pumpe vornehmen, deren Antriebsmotor von einem die Menge des gesamten Abwassers messenden Durchflußmengenmesser gesteuert wird.

Das abgezweigte Abwasser kann in einem mit einem Rührwerk versehenen Behälter gesammelt werden, wobei vor der Bestimmung der Schadstoffe in dem gesammelten Abwasser mittels üblicher Analyseverfahren dieses gleichmäßig verrührt wird, um zuverlässige Durchschnittswerte bei der Schadstoffbestimmung zu erhalten.

Eine zur Durchführung des vorbeschriebenen Verfahrens geeignete Vorrichtung kennzeichnet sich durch einen einer vom gesamten Abwasser durchströmten Ableitung zugeordneten Durchflußmengenmesser und durch einen mit dieser Ableitung über eine Abzweigleitung verbundenen Sammelbehälter, wobei in der Abzweigleitung eine Pumpe angeordnet ist, deren steuerbarer Antriebsmotor mit dem Durchflußmengenmesser zwecks Steuerung bzw. Einstellung einer bestimmten Fördermenge in der Abzweigleitung verbunden ist.

Vorteilhaft ist es dabei, die Pumpe in der Abzweigleitung als Membranpumpe auszubilden, da ein Verstellmotor für eine derartige Membranpumpe sich zu einer Steuerung mittels eines Durchflußmengenmessers besonders gut eignet.

Der Sammelbehälter kann vorzugsweise mit einem Rührwerk versehen sein.

Schließlich empfiehlt es sich, den Durchflußmengenmesser, die Abzweigleitung mit Pumpe und zugeordnetem Antrieb und den Sammelbehälter in einem plombierbaren Gehäuse anzuordnen, um Manipulationen an den betreffenden Vorrichtungsteilen auszuschließen.

Die Erfindung ist nachstehend anhand der Zeichnung beispielsweise näher erläutert. Diese zeigt in schematischer Darstellung eine Vorrichtung gemäß der Erfindung.

Von einem vom gesamten Abwasser durchströmten Behälter 1 führt eine Ableitung 2 zu einem Abwasserkanal 3 oder dgl. Von der Ableitung 2 führt eine Abzweigleitung 4 zu einem Sammelbehälter 5. In der Abzweigleitung 4 ist eine Membranpumpe 6 angeordnet, deren Verstellmotor 7 mit einem Durchflußmengenmesser 8 verbunden ist, der in der Ableitung 2 angeordnet bzw. dieser zugeordnet ist.

Im Sammelbehälter 5 ist ferner noch ein Rührwerk 9 angeordnet.

Der Durchflußmengenmesser 8, die Abzweigleitung 4 mit Pumpe 6 und Antrib 7 sowie der Sammelbehälter 5 sind in einem plombierbaren, gestrichelt angedeuteten Gehäuse 10 angeordnet, so daß die betreffenden Teile nur für Kontrollpersonen der zuständigen Abwasserbehörde zugänglich sind.

Die Wirkungsweise der Vorrichtung ist wie folgt:

In dem Behälter 1 sammelt sich das aus einer bestimmten Anlage herrührende Abwasser, wobei bei der Verwendung von Säuren, wie diese z.B. in Eloxier-Anlagen Anwendung finden, basische Mittel zur Neutralisation der Säuren zugegeben worden sind. Aus dem Behälter 1 strömt das Abwasser durch die Ableitung 2, entweder durch Schwerkraft oder mittels einer nicht dargestellten Pumpe zu einem Abwasserkanal 3. Das durch die Ableitung 2 passierende Abwasser wird vom Durchflußmengenmesser 8 kontinuierlich gemessen und eine entsprechende Meßkenngröße wird dem Verstellmotor 7 für die Membranpumpe 6 derart aufgegeben, daß ein bestimmter Anteil der die Ableitung 2 passierenden Abwassermenge, beispielsweise ein Tausendstel, über die Abzweigleitung 4 dem Sammelbehälter 5 zugeführt wird. Würde beispielsweise in der betreffenden Anlage eine Abwassermenge von 30 m³/Tag anfallen, so würden über die Abzweigleitung 4 30l Wasser in den Sammelbehälter 5 gelangen. Ein Sammelbehälter von 3 m³ Fassungsvermögen könnte dann das die Abzweigung 4 passierende Sammelwasser von etwa 100 Betriebstagen aufnehmen, d.h. eine Messung bzw. Analyse der Schadstoffe des im Sammelbehälter 5 angsammelten Abwassers brauchte nur ca. 2 mal im Jahr zu erfolgen, um praktisch genaue Angaben über die Menge der die betreffende Anlage verlassenden Abwässer und die in diesen enthaltenen Schadstoffe zu erhalten. Um bei der Probenentnahme aus dem Sammelbehälter 5 keine verfälschten Ergebnisse zu erhalten, wird kurz vor der Probenentnahme das Rührwerk 9 in Betrieb gesetzt. Nach der Probenentnahme kann dann der Sammelbehälter durch entsprechendes Umschalten der Membranpumpe 6 in den Kanal 3 entleert werden, so daß der Sammelbehälter 5 für einen neuen Arbeitszeitraum wieder zur Verfügung steht.

Natürlich kann man bei entsprechenden praktischen Bedürfnissen den Anteil der aus der Gesamtabwassermenge abgezweigten und im Sammelbehälter 5 gesammelten Abwassermenge auch wesentlich erhöhen, beispielsweise im Verhältnis 1 : 100, wenn dies aus bestimmten Gründen zweckmäßig oder empfehlenswert ist, wodurch sich die Meßergebnisse natürlich noch verbessern.

Das beschriebene Ausführungsbeispiel ist in vielfacher Weise abzuändern, ohne den Grundgedanken der Erfindung zu verlassen. So könnte beispielsweise bei drucklosem und annähernd konstantem Ablaufen des Abwassers aus dem Behälter 1 evtl. auch auf eine Pumpe in der Abzweigleitung 4 verzichtet werden, indem man den Querschnitt der Abzweigleitung 4 zum Querschnitt der Ableitung 2 derart bemißt, daß ein experimentell zu ermittelnder Teil der gesamten Abwassermenge aus der Ableitung 2 über die Abzweigleitung 4 in den Sammelbehälter 5 fließt, so daß man dann aus der schließlich im Sammelbehälter 5 sich angesammelt habenden Menge sowohl die gesamte Abwassermenge errechnen kann als auch in der zuvor beschriebenen Weise durch Analyse der Schadstoffe im Abwassersammelbehälter 5 auf die gesamten Schadstoffmengen rückschließen kann und dgl. mehr.

## Ansprüche

1. Verfahren zur Messung von Schadstoffen in Abwässern,
dadurch gekennzeichnet,
daß die Menge des gesamten Abwassers kontinuierlich gemessen und ein bestimmter Teil des jeweils abgeführten Abwassers abgezweigt und gesammelt wird, worauf die Schadstoffmengen in dem abgezweigten und gesammelten Abwasser bestimmt werden und entsprechend dem Verhältnis der Menge des abgezweigten und gesammelten Abwassers zur Menge des gesamten Abwassers die Schadstoffmengen im gesamten Abwasser ermittelt bzw. hochgerechnet werden.

2. Verfahren nach Anspruch 1,
dadurch gekennzeichnet,
daß die Abzweigung der Menge des zu sammelnden Abwassers etwa in einem Verhältnis von 1 : 1000 zur Menge des gesamten Abwassers erfolgt.

3. Verfahren nach Anspruch 1 oder 2,
dadurch gekennzeichnet,
daß die Abzweigung des zu sammelnden Abwassers durch eine Pumpe erfolgt, deren Antriebsmotor von einem die Menge des gesamten Abwassers messenden Durchflußmengenmesser gesteuert wird.

4. Verfahren nach Anspruch 1 oder einem der folgenden,
dadurch gekennzeichnet,
daß das abgezweigte Abwasser in einem mit einem Rührwerk versehenen Behälter gesammelt wird, wobei vor der Bestimmung der Schadstoffe in dem gesammelten Abwasser dieses gleichmäßig verrührt wird.

5. Vorrichtung zur Durchführung des Verfahrens nach Anspruch 1 oder einem der folgenden, gekennzeichnet durch,
einen einer vom gesamten Abwasser durchströmten Ableitung (2) zugeordneten Durchflußmengenmesser (8) und durch einen mit dieser Ableitung über eine Abzweigleitung (4) verbundenen Sammelbehälter (5), wobei in der Abzweigleitung (4) eine Pumpe (6) angeordnet ist, deren steuerbarer Antriebsmotor (7) mit dem Durchflußmengenmesser (8) zwecks Steuerung bzw. Einstellung einer bestimmten Fördermenge in der Abzweigleitung verbunden ist.

6. Vorrichtung nach Anspruch 5,
dadurch gekennzeichnet,
daß die Pumpe in der Abzweigleitung als Membranpumpe (6) ausgebildet ist.

7. Vorrichtung nach Anspruch 5 oder 6,
dadurch gekennzeichnet,
daß der Sammelbehälter (5) mit einem Rührwerk (9) versehen ist.

8. Vorrichtung nach Anspruch 5 oder einem der folgenden,
dadurch gekennzeichnet,
daß der Durchflußmengenmesser (8), die Abzweigleitung (4) mit Pumpe (6) und zugeordnetem Antrieb (7) und der Sammelbehälter (5) in einem plombierbaren Gehäuse (10) angeordnet sind.